(19) Europäisches Patentamt
European Patent Office
Office européen des brevets

(11) Veröffentlichungsnummer: **0 251 014 B1**

(12) **EUROPÄISCHE PATENTSCHRIFT**

(45) Veröffentlichungstag der Patentschrift: **20.05.92**

(51) Int. Cl.5: **C07D 235/10**, C07D 491/04, A01N 43/52, A01N 43/90

(21) Anmeldenummer: **87108593.2**

(22) Anmeldetag: **15.06.87**

Die Akte enthält technische Angaben, die nach dem Eingang der Anmeldung eingereicht wurden und die nicht in dieser Patentschrift enthalten sind.

# (54) 2-Trifluormethyl-benzimidazole.

(30) Priorität: **25.06.86 DE 3621301**

(43) Veröffentlichungstag der Anmeldung:
**07.01.88 Patentblatt 88/01**

(45) Bekanntmachung des Hinweises auf die Patenterteilung:
**20.05.92 Patentblatt 92/21**

(84) Benannte Vertragsstaaten:
**AT BE CH DE FR GB IT LI NL SE**

(56) Entgegenhaltungen:
**DE-A- 1 620 370** **DE-A- 1 642 334**
**FR-A- 2 007 808** **GB-A- 1 202 874**
**US-A- 3 632 397** **US-A- 4 122 184**

**Z. Naturforsch., 256, S.934-953,1970**

(73) Patentinhaber: **BAYER AG**

**W-5090 Leverkusen 1 Bayerwerk(DE)**

(72) Erfinder: **Heywang, Gerhard, Dr.**
**Nittumer Weg 4**
**W-5060 Bergisch-Glagbach 2(DE)**
Erfinder: **Baasner, Bernd, Dr.**
**Hamberger Strasse 27d**
**W-5090 Leverkusen 3(DE)**
Erfinder: **Marhold, Albrecht, Dr.**
**Carl-Duisberg Strasse 329**
**W-5090 Leverkusen 1(DE)**
Erfinder: **Kysela, Ernst, Dr.**
**Virchowstrasse 14**
**W-5060 Bergisch Gladbach 3(DE)**
Erfinder: **Schwamborn, Michael, Dr.**
**von-Lohe-Strasse 9**
**W-5000 Köln 80(DE)**
Erfinder: **Hänssler, Gerd, Dr.**
**Am Arenzberg 58a**
**W-5090 Leverkusen 3(DE)**
Erfinder: **Paulus, Wilfried, Dr.**
**Deswatinestrasse 90**
**W-4150 Krefeld(DE)**
Erfinder: **Santel, Hans-Joachim, Dr.**
**Grünstrasse 9a**
**W-5090 Leverkusen 1(DE)**
Erfinder: **Schmidt, Robert R, Dr.**
**Im Waldwinkel 110**
**W-5060 Bergisch-Gladbach 2(DE)**
Erfinder: **Schmitt, Hans-Georg, Dr.**
**Am Oberend 13**
**W-4150 Krefeld 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft neue 2-Trifluormethylbenzimidazole, ein Verfahren zu ihrer Herstellung sowie ihre Verwendung als Herbizide und Mikrobizide.

Es ist bereits bekannt, daß bestimmte 2-Trifluormethylbenzimidazole herbizide, mikrobizide, fungizide und z.T. auch insektizide Eigenschaften aufweisen [vergl. z.B. DE-OS 1 642 334, DE-AS 2 150 219, Pestic Sci. 15, 31 (1984), J. med. Chem. 13, 1043 (1970), Z. Naturforsch. 256, 934 und 945 (1970)]. Die Wirkung dieser Verbindungen ist jedoch in bestimmten Indikationsgebieten unter bestimmten Bedingungen, z.B. bei niedrigen Aufwandmengen und -konzentrationen, nicht immer befriedigend.

Es wurden nun neue 2-Trifluormethyl-benzimidazole der allgemeinen Formel (I) gefunden,

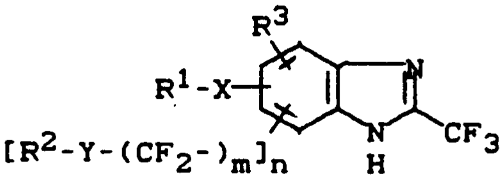

(I)

in welcher

| | |
|---|---|
| $R^1$ | für Halogenalkyl steht, |
| $R^2$ | für gegebenenfalls durch Fluor-, Chlor- oder Brom substituiertes Alkyl steht oder |
| $R^1$ und $R^2$ | gemeinsam für gegebenenfalls durch Fluor-, Chlor- oder Brom substituiertes Alkylen stehen |
| $R^3$ | für Wasserstoff oder Alkyl steht, |
| X und Y | unabhängig voneinander für Sauerstoff und Schwefel stehen, |
| m | für 0 oder 1 steht und |
| n | für 0 oder 1 steht. |

Weiterhin wurde gefunden, daß man die neuen 2-Trifluormethyl-benzimidazole der Formel (I),

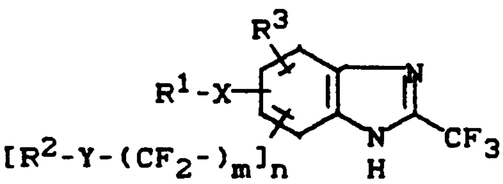

(I)

in welcher

| | |
|---|---|
| $R^1$ | für Halogenalkyl steht, |
| $R^2$ | für gegebenenfalls durch Fluor-, Chlor- oder Brom substituiertes Alkyl steht oder |
| $R^1$ und $R^2$ | gemeinsam für gegebenenfalls durch Fluor-, Chlor- oder Brom substituiertes Alkylen stehen |
| $R^3$ | für Wasserstoff oder Alkyl steht, |
| X und Y | unabhängig voneinander für Sauerstoff und Schwefel stehen, |
| m | für 0 oder 1 steht und |
| n | für 0 oder 1 steht, |

erhält, wenn man o-Phenylendiamine der Formel (II),

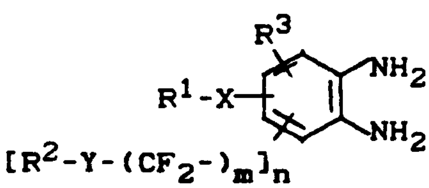

(II)

in welcher

$R^1$, $R^2$, $R^3$, X, Y, m und n die oben angegebene Bedeutung haben,

mit Trifluoressigsäure gegebenenfalls in Gegenwart von konzentrierter Salzsäure und gegebenenfalls in Gegenwart eines Verdünnungsmittels umsetzt.

Schließlich wurde gefunden, daß die neuen 2-Trifluormethyl-benzimidazole der Formel (I) herbizide, insbesondere auch selektiv-herbizide, mikrobizide und fungizide Eigenschaften besitzen.

Überraschenderweise zeigen die erfindungsgemäßen 2-Trifluormethyl-benzimidazole der Formel (I) neben einer besseren herbiziden Wirksamkeit gegen Unkräuter auch eine ausgezeichnete Nutzpflanzenverträglichkeit und zeigen darüberhinaus eine bessere mikrobizide und fungizide Wirksamkeit als die aus dem Stand der Technik bekannten Benzimidazole gleicher Wirkungsart.

Im Rahmen der obigen Substituentendefinitionen steht Alkyl vorzugsweise für einen geradkettigen oder verzweigten gesättigten Kohlenwasserstoff mit 1 bis 6 Kohlenstoffatomen, besonders bevorzugt mit 1 bis 5 Kohlenstoffatomen, wie beispielsweise Methyl, Ethyl, n- und i-Propyl, n-, i-, s- und t-Butyl, n- und i-Pentyl.

Halogenalkyl steht vorzugsweise für einen geradkettigen oder verzweigten gesättigten Kohlenwasserstoff mit 1 bis 6 Kohlenstoffatomen und 1 bis 13 gleichen oder verschiedenen Halogenatomen, besonders bevorzugt mit 1 bis 4 Kohlenstoffatomen und 1 bis 9 gleichen oder verschiedenen Halogenatomen (wie Fluor, Chlor, Brom), insbesondere mit 1 bis 2 Kohlenstoffatomen und 1 bis 5 gleichen oder verschiedenen Halogenatomen (wie Fluor und Chlor) wie beispielsweise Trifluormethyl, Trichlormethyl, Trifluorchlormethyl und Trifluorethyl.

Alkylen steht vorzugsweise für einen geradkettigen oder verzweigten gesättigten Kohlenwasserstoff mit 1 bis 4 Kohlenstoffatomen, besonders bevorzugt mit 1 oder 2 Kohlenstoffatomen, wie beispielsweise Methylen und Ethylen.

Halogen steht für Fluor, Chlor, Brom und Iod, bevorzugt für Fluor, Chlor und Brom und insbesondere für Fluor und Chlor.

Die erfindungsgemäßen 2-Trifluormethyl-benzimidazole sind durch die Formel (I) allgemein definiert. Bevorzugt sind die Verbindungen der Formel (I), bei welchen

| | |
|---|---|
| $R^1$ | für Halogenalkyl mit 1 bis 6 Kohlenstoffatomen und 1 bis 13 gleichen oder verschiedenen Halogenatomen steht, |
| $R^2$ | für gegebenenfalls ein- oder mehrfach, gleich oder verschieden durch Fluor, Chlor und Brom substituiertes Alkyl mit 1 bis 6 Kohlenstoffatomen steht oder |
| $R^1$ und $R^2$ | gemeinsam für gegebenenfalls ein- oder mehrfach, gleich oder verschieden durch Fluor, Chlor und Brom substituiertes Alkylen mit 1 bis 4 Kohlenstoffatomen stehen, |
| $R^3$ | für Wasserstoff oder Alkyl mit 1 bis 6 Kohlenstoffatomen steht, |
| X und Y | unabhängig voneinander für Sauerstoff und Schwefel stehen, |
| m | für 0 oder 1 steht und |
| n | für 0 oder 1 steht. |

Besonders bevorzugt sind diejenigen 2-Trifluormethylbenzimidazole der Formel (I), bei welchen

| | |
|---|---|
| $R^1$ | für Halognalkyl mit 1 bis 4 Kohlenstoffatomen und 1 bis 9 gleichen oder verschiedenen Fluor-, Chlor- oder Bromatomen steht, |
| $R^2$ | für gegebenenfalls ein- bis neunfach, gleich oder verschieden durch Fluor und Chlor substituiertes Alkyl mit 1 bis 4 Kohlenstoffatomen steht oder |
| $R^1$ und $R^2$ | gemeinsam für gegebenenfalls einfach bis fünffach, gleich oder verschieden durch Fluor und Chlor substituiertes Alkylen mit 1 bis 4 Kohlenstoffatomen stehen, |
| $R^3$ | für Wasserstoff oder Alkyl mit 1 bis 4 Kohlenstoffatomen steht, |
| X und Y | unabhängig voneinander für Sauerstoff und Schwefel stehen, |
| m | für 0 oder 1 steht und |
| n | für 0 oder 1 steht. |

Ganz besonders bevorzugt sind die Verbindungen der Formel (I), in denen

| | |
|---|---|
| $R^1$ | für Fluormethyl, Difluormethyl, Trifluormethyl, Chlormethyl, Dichlormethyl, Trichlormethyl, Chlordifluormethyl, Dichlorfluormethyl, 1.1.2.2-Tetrafluorethyl, 2-Chlor-1.1.2-trifluorethyl, 2.2.2-Trifluorethyl, 2.2-Dichlor-1.1.2-trifluorethyl, 2-Chlor-1.1.2.2-tetrafluorethyl, 2-Chlor-2.2-difluorethyl, 2.2-Dichlor-2-fluorethyl, 2.2.2-Trichlorethyl, 1.1.2.3.3.-Hexafluorpropyl steht, |
| $R^2$ | für Fluormethyl, Difluormethyl, Trifluormethyl, Chlormethyl, Dichlormethyl, Trichlormethyl, Chlordifluormethyl, Dichlorfluormethyl, 1.1.2.2-Tetrafluorethyl, 2-Chlor-1.1.2-trifluorethyl, 2.2.2-Trifluorethyl, 2.2-Dichlor-1.1.2-trifluorethyl, 2-Chlor-1.1.2.2-tetrafluorethyl, 2-Chlor-2.2-difluorethyl, 2.2-Dichlor-2-fluorethyl, 2.2.2-Trichlorethyl, 1.1.2.3.3.-Hexafluorpropyl steht oder |
| $R^1$ und $R^2$ | zusammen für Difluormethylen, Chlorfluormethylen, $\alpha,\beta$-Difluorethylen, $\alpha,\alpha$-Difluorethylen, Trifluorethylen, Tetrafluorethylen, Chlortrifluorethylen, Chlordifluorethylen, Chlorfluorethylen oder Dichlorfluorethylen stehen, |
| $R^3$ | für Wasserstoff, Methyl, Ethyl, Propyl und iso-Propyl steht und |

X, Y, m und n die oben angegebenen Bedeutungen haben.

Beispielhaft seien die folgenden neuen 2-Trifluormethylbenzimidazole der Formel (I) namentlich aufgeführt:

4-Trifluormethoxy-2-trifluormethyl-benzimidazol
7-Trifluormethoxy-2-trifluormethyl-benzimidazol
5-Trifluormethoxy-2-trifluormethyl-benzimidazol
6-Trifluormethoxy-2-trifluormethyl-benzimidazol
4-Trifluormeththio-2-trifluormethyl-benzimidazol
5-Trifluormeththio-2-trifluormethyl-benzimidazol
4-[2,2,2-Trifluorethoxy]-2-trifluormethyl-benzimidazol
5-[2,2,2-Trifluorethoxy]-2-trifluormethyl-benzimidazol
4-[1,1,2,2-Tetrafluorethoxy]-2-trifluormethyl-benzimidazol
4-[2-Chlor-1,1,2-trifluorethoxy]-2-trifluormethyl-benzimidazol
5-[1,1,2,2-Tetrafluorethoxy]-2-trifluormethyl-benzimidazol
5-[2-Chlor-1,1,2-trifluorethoxy]-2-trifluormethyl-benzimidazol
4,5-Bistrifluormethoxy-2-trifluormethyl-benzimidazol
5,6-Bistrifluormethoxy-2-trifluormethyl-benzimidazol
4,6-Bistrifluormethoxy-2-trifluormethyl-benzimidazol
5-Methoxy-6-trifluormethoxy-2-trifluormethyl-benzimidazol
5,6-Difluormethylen-dioxo-2-trifluormethyl-benzimidazol
5,6-Trifluorethylen-dioxo-2-trifluormethyl-benzimidazol
5,6-Tetrafluorethylen-dioxo-2-trifluormethyl-benzimidazol
5,6-Chlortrifluorethylen-dioxo-2-trifluormethyl-benzimidazol
6-[2-Chlor-1,1,2-trifluorethoxy]-5-methyl-2-trifluormethyl-benzimidazol
5-[2-Chlor-1,1,2-trifluormethoxy]-6-methyl-2-trifluormethyl-benzimidazol
7,7,9,9-Tetrafluor-6,7,8,9-tetrahydro-2-trifluormethyl-6,8-dioxabenzo[g]benzimidazol.

Verwendet man beispielsweise 2-Trifluormethoxy-5,6-diamino-benzol und Trifluoressigsäure als Ausgangsstoffe, so läßt sich der Reaktionsablauf des erfindungsgemäßen Verfahrens durch das folgende Formelschema wiedergeben:

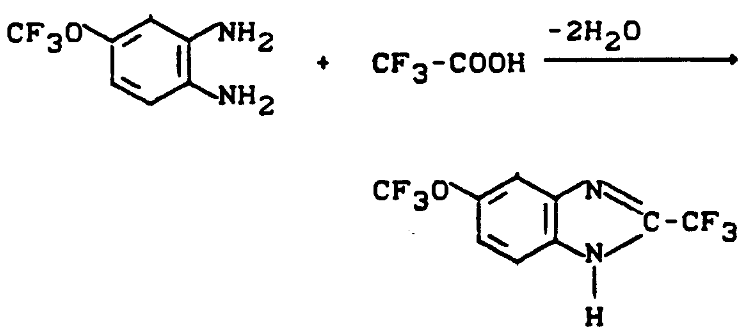


Die zur Durchführung des erfindungsgemäßen Verfahrens benötigen o-Phenylendiamine durch die Formel (II) allgemein definiert. $R^1$, $R^2$, $R^3$, X, Y, m und n haben darin die Bedeutung, die bereits im Zusammenhang mit der Beschreibung der erfindungsgemäßen Stoffe der Formel (I) für diese Symbole genannt wurde.

Die Ausgangsstoffe der Formel (II) sind teilweise bekannt (vgl. z.B. EP-A-127,763). Die noch nicht bekannten o-Phenylendiamine der Formel (II) sind teilweise Gegenstand der nicht vorveröffentlichten Patentanmeldung DE-P 36 05 977 vom 25. Februar 1986 und teilweise einer parallel zu dieser Anmeldung eingereichten deutschen Patentanmeldung.

Die neuen und auch die bekannten Verbindungen der allgemeinen Formel (II) können hergestellt werden (vgl. z.B. L.M. Yagupolskij et al., Zh. Obsh. Khim 33, 3051-5 (1963); Houben-Weyl, Band X/1, S. 559 (1971) und Band XI/1, S. 472-3 (1957) und Herstellungsbeispiele), indem man beispielsweise Verbindungen der Formel (III)

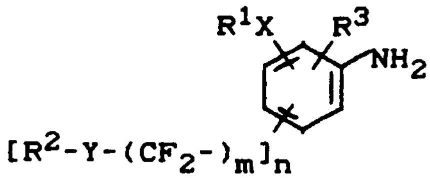

(III)

in der

$R^1$, $R^2$, $R^3$, X, Y, m und n die oben angegebene Bedeutung haben,

zunächst an der Aminogruppe acyliert, dann in 2-Stellung zur acylierten Aminogruppe mit einem Nitrierungsmittel wie beispielsweise Nitriersäure, gegebenenfalls in Gegenwart eines Verdünnungsmittels, wie beispielsweise Eisessig, und gegebenenfalls in Gegenwart eines Katalysators wie beispielsweise Acetanhydrid, bei Temperaturen zwischen -20 und +50°C eine Nitrogruppe einführt, die Nitrogruppe in Gegenwart eines Katalysators, wie beispielsweise Raney-Nickel, und in Gegenwart eines Verdünnungsmittels, wie beispielsweise Methanol bei Wasserstoffdrucken von 10 bis 100 bar und bei Temperaturen zwischen +20 und +80°C zur Aminogruppe hydriert und dann die Acylgruppe in üblicher Weise, beispielsweise durch Verseifung mit wäßriger oder alkoholischer Base, wieder abspaltet.

Die Verbindungen der Formel (III) sind bekannt und lassen sich nach bekannten Verfahren in analoger Weise herstellen (vgl. z.B. EP 11 179).

Die zur Durchführung des erfindungsgemäßen Verfahrens weiterhin benötigte Trifluoressigsäure ist eine bekannte Verbindung der organischen Chemie.

Als Verdünnungsmittel zur Durchführung des erfindungsgemäßen Verfahrens kommen inerte organische Lösungsmittel, beispielsweise Toluol, Chlorbenzol und Dichlorbenzol infrage. Bevorzugt verwendet man Toluol.

Die Reaktion kann auch in konz. wäßriger Salzsäure gemäß M.A. Phillips, J. Chem. Soc. 1928, 2393, durchgeführt werden.

Die Reaktionstemperaturen können bei der Durchführung des erfindungsgemäßen Verfahrens in einem größeren Bereich variiert werden. Im allgemeinen arbeitet man bei Temperaturen zwischen 20°C und 220°C, vorzugsweise zwischen 20°C und 160°C bzw. am Siedepunkt der jeweils am niedrigsten siedenden Komponente der im Reaktionsgemisch vorhandenen Komponenten.

Das erfindungsgemäße Verfahren wird im allgemeinen bei Normaldruck durchgeführt. Es kann aber auch bei erhöhtem oder vermindertem Druck gearbeitet werden.

Zur Durchführung des erfindungsgemäßen Verfahrens setzt man pro Mol o-Phenylendiamin der Formel (II) im allgemeinen 1 bis 20 Mol, vorzugsweise 1 bis 10 Mol, besonders bevorzugt 1,1 bis 5 Mol Trifluoressigsäure ein.

Die o-Phenylendiamine der Formel (II) können in dem erfindungsgemäßen Verfahren auch in Form ihrer Salze, gebildet aus (II) und einer geeigneten organischen Säure, wie z.B. Essigsäure oder auch als Hydrochloride eingesetzt werden. Auch die acylierten Diamine können zur Durchführung des erfindungsgemäßen Verfahrens eingesetzt werden.

Die Aufarbeitung, Isolierung und Charakterisierung der 2-Trifluormethyl-benzimidazole der Formel (I) erfolgen nach allgemein üblichen Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hofpenanlagen und zur selektiven Unkrautbekämpfung in einjährigen Kulturen eingesetzt werden.

Dabei zeigen die erfindungsgemäßen Stoffe der Formel (I) neben einer besonders guten allgemeinherbiziden Wirksamkeit auch eine deutlich verbesserte Kulturpflanzenselektivität in wichtigen Kulturen und können als selektive Unkrautbekämpfungsmittel, insbesondere gegen dikotyle Unkräuter, in dikotylen Kulturen, wie beispielsweise Baumwolle, als auch in monokotylen, Kulturen, insbesondere Getreide, wie beispielsweise Weizen, im Vor- und im Nachauflaufverfahren eingesetzt werden. Insbesondere im Nachauflaufverfahren eignen sich die erfindungsgemäßen Verbindungen der Formel (I) auch zur Bekämpfung monokotyler Unkräuter.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspension-Emulsion-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage; Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
Z.B. Ammoniumsalze und natürliche Gesteinsmehle, wie Kaloine, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmorm Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarbicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten bei der Anwendung als Herbizid und Fungizid im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Umkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N-dimethyl-harnstoff zur Unkrautbekämpfung in Getreide; 4-Amino-3-methy-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit Chloressigsäure-N-(methoxymethyl)-2,6-diethylanilid, 2-Ethyl-6-methyl-N-(1-Methyl-2-methoxyethyl)-chloracetanilid, N-Methyl-2-(1,3-benzthiazol-2-yloxy)-actanilid, N,N-Diisopropyl-S-(2,3,3-trichlorallyl)-thiolcarbamat, N,N-Di-n-propyl-thiocarbamidsäure-S-ethylester, exo-1-Methyl-4-(1-methylethyl)-2-(2-methyl-phenylmethoxy)-7-oxabicyclo-[2.2.1]-heptan, 1-Methyl-3-phenyl-5-[3-trifluormethyl-phenyl]-4(1H)-pyridinon, Methyl-2-[4,5-dihydro-4-methyl-4-(1-methylethyl)-5-oxo-1H-imidazol-2-yl]-4(5)-methylbenzoat, 2-[1-Ethoxamino)-butyliden]-5-(2-ethylthiopropyl)-1,3-cyclohexandion,3-Isopropyl-2,1,3-benzothiadiazin-4-on-2,2-dioxid, 6-Chlor-3-phenyl-pyridazin-4-yl-S-octyl-thiocarbonat, N-Benzthiazolyl-N-methyl-N′-(3-chlor-4-methylphenyl)-harnstoff, 2-Chlor-N-{[(4-methoxy-6-methyl-1,3,5-triazin-2-yl)-amino]-carbonyl}-benzolsulfonamid, N,N-Dimethyl-N′-(4-isopropylphenyl)-harnstoff, N,N-Dimethyl-N′-(3-trifluormethyl-phenyl)-harnstoff, 4-Ethylamino-2-t-butylamino-6-methylthio-s-triazin, 2-Chlor-4,6-bis-(ethylamino-1,3,5-triazin, 2-Chlor-4-ethylamino-6-isopropylamino-1,3,5-triazin, 2-Chlor-4-ethylamino-6-(3-cyanopropylamino)-1,3,5-triazin, 4-Amino-6-tert.-butyl-3-methylthio-4,5-dihydro-1,2,4-triazin-5-on, 2-[4,$\beta$,5-Dichlorpyrid-2-yloxy)-phenoxy]-propionsäure-trimethylsilylmethylester, 2-{4-[(3-Chlor-5-trifluormethyl-2-pyridyl)-oxy]-phenoxy}-propansäure, 2-[4-(2,4-Dichlorphenoxy)-phenoxy]-propionsäuremethylester, 2,4-Dichlorphenoxyessigsäure, 2,4-Dichlorphenoxy-propionsäure, (2-Methyl-4-chlorphenoxy)-essigsäure, (4-Chlor-2-methyl-phenoxy)-propionsäure, Methyl-5-(2,4-dichlorphenoxy)-2-nitrobenzoat, 3,5-Diiod-4-hydroxybenzonitril, 3,5-Dibrom-4-hydroxybenzonitril, N-(1-Ethoxypropyl)-3,4-dimethyl-2,6-dinitroanilin kommen in Frage, Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann bei der Anwendung als Herbizid in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die 2-Trifluormethyl-benzimidazole der Formel (I) weisen auch eine starke mikrobizide Wirkung auf und können zur Bekämpfung von unerwünschten Mikroorganismen eingesetzt werden. Die Wirkstoffe sind für den Gebrauch als Pflanzenschutzmittel, insbesondere als Fungizide, geeignet oder im Materialschutz zum Schutz technischer Materialien einsetzbar.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podospharea leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);

Cochliobolus-Arten, wie beispielsweise Cochliobolus sativus (Konidienform: Drechslera, Syn: Helminthosproium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Bei der Behandlung von Pflanzenteilen können die Wirkstoffkonzentrationen bei der Anwendung als Fungizid in den Anwendungsformen in einem größeren Bereich variiert werden. Sie liegen im allgemeinen zwichen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Darüberhinaus weisen die erfindungsgemäßen Wirkstoffe der Formel (I) eine gute Wirksamkeit bei der Bekämpfung von Bodeninsekten und Nematoden auf.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Kelbstoffe, Leime, Papier und Karton, Textilein, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikrooganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten), sowie gegen Bakterien, Schleimorganismen und Algen.
Es seien beispielsweise Mikrooganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporous, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeroginosa,
Staphylococcus, wie Staphylococcus aureus.

Je nach Anwendungsgebiet kann ein erfindungsgemäßer Wirkstoff in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls

unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohle, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Mikrobizide Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen Wirkstoffe können auch in Mischung mit anderen bekannten Wirkstoffen vorliegen. Beispielsweise seien die folgenden Wirkstoffe genannt: Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenthiazol, 2-Rhodanidomethylthiobenzthiazol, Organo-Zinnverbindungen, Methylenbisthiocyanat, Phenolderivate, wie 2-Phenylphenol, (2,2'-Dihydroxy-5,5'-dichlor)diphenylmethan und 3-Methyl-4-chlor-phenol.

Die Herstellung und die Verwendung der erfindungsgemäßen Wirkstoffe geht aus den nachfolgenden Beispielen hervor.

Herstellungsbeispiele

Beispiel 1

2-Trifluormethyl-5,5,6,6-tetrafluorethylendioxobenzimidazol

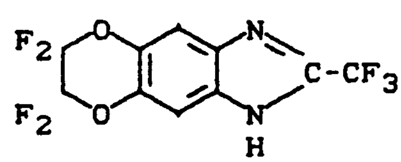

Zu 23,8 g (0,1 Mol) 2,2,3,3-Tetrafluor-6,7-Diamino-benzo-1,4-dioxen werden in ca. 20 min 45 g (0,39 Mol) Trifluoressigsäure getropft. Die Temperatur steigt von 20 °C auf etwa 60 °C an, es wird nicht gekühlt. Anschließend wird in ca. 30 min auf 130 °C (Badtemperatur) aufgeheizt und 2 h bei dieser Temperatur nachgerüht. Nach Erkalten wird das Gemisch durch Zutropfen von 120 ml 10 %iger Natronlauge alkalisch gestellt und der ausgefallene Feststoff abgesaugt, mit Wasser nachgewaschen und getrocknet.
Ausbeute: 28,1 g (88,8 % der Theorie)
Schmelzpunkt: 235-237 °C

In analoger Weise erhält man die in der folgenden Tabelle 1 aufgeführten Verbindungen der Formel (I):

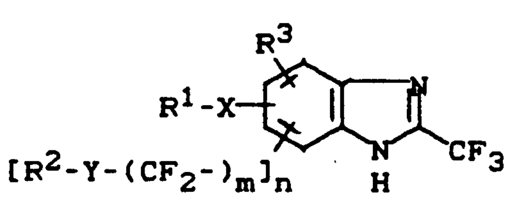
(I)

Tabelle 1

| Beispiel Nr. | Verbindung | Fp. (°C) |
|---|---|---|
| 2 | 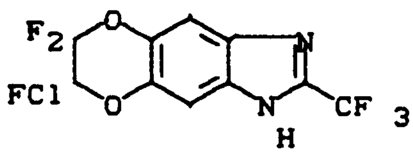 | 203 - 205 |
| 3 | 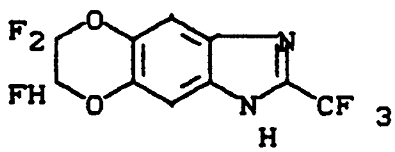 | 257 - 259 |
| 4 | 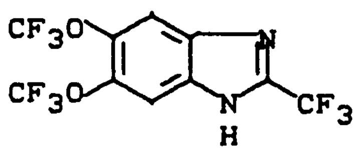 | 157-158 |
| 5 | 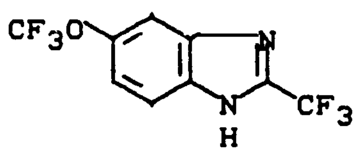 | 167-168 |
| 6 | 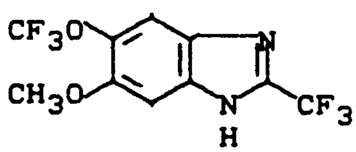 | 155-156 |
| 7 | 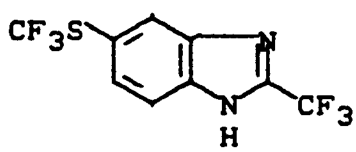 | 175-176 |
| 8 | 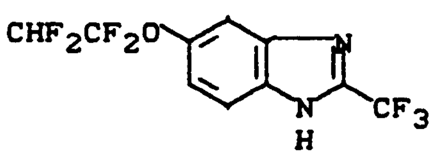 | 188-189 |
| 9 | 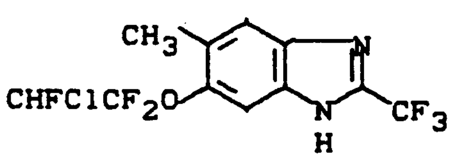 | 158-160 |

**Tabelle 1 (Fortsetzung)**

| Beispiel Nr. | Verbindung | Fp. (°C) |
|---|---|---|
| 10 | 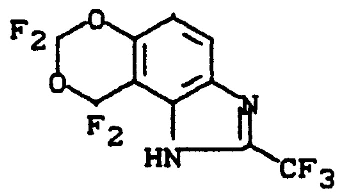 | 235-237 |

Herstellung der Ausgangsprodukte:

Beispiel II-1

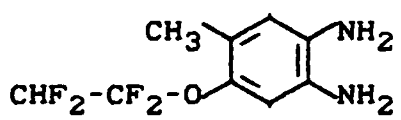

(II-1)

a) Acylierung

114 g (0,51 Mol) 4-Methyl-5-(1,1,2,2-tetrafluorethoxy)anilin werden in eine 50 °C warme Lösung aus 60 g Acetanhydrid, 20 g Essigsäure und 2 ml Pyridin getropft. Nach zweistündigem Nachrühren bei 50 °C wird wäßrig aufgearbeitet. Man erhält 132 g 4-Methyl-5-(1,1,2,2-tetrafluorethoxy)acetanilid (Fp:124 °C).

b) Nitrierung

132 g (0,5 Mol) 4-Methyl-5-(1,1,2,2-tetrafluorethoxy)-acetanilid werden unter Zusatz von 165 g Nitriersäure (33 Gew.-% $HNO_3$, 67 Gew.-% $H_2SO_4$) und 25 g Wasser bei 0 bis 5 °C nitriert. Nach wäßriger Aufarbeitung werden 110 g rohes 2-Nitro-4-methyl-5-(1,1,2,2-tetrafluorethoxy)-acetanilid erhalten.

c) Hydrierung

Das nitrierte Produkt wird in 250 ml Methanol unter Zusatz von 10 g Raney-Nickel bei 50 °C und 30-50 bar Wasserstoffdruck hydriert. Nach Abtrennen des Raney-Nickels und des Methanols werden 90 g rohes 2-Amino-4-methyl-5-(1,1,2,2-tetrafluorethoxy)-acetanilid erhalten.

d) Verseifung

Das hydrierte Produkt wird mit 350 ml Methanol und 100 g 50 gew.%iger wäßriger Natronlager versetzt, die Mischung 5 Stunden bei 45 °C und weiter 7 Stunden bei 25 °C gerührt. Nach wäßriger Aufarbeitung werden 68 g 2-Amino-4-methyl-5-(1,1,2,2-tetrafluorethoxy)-anilin mit einem Siedepunkt von 105-107 °C/0,04 mbar und einem Schmelzpunkt von 99-103 °C erhalten.

In analoger Weise erhält man die in der folgenden Tabelle 2 aufgeführten o-Phenylendiamine der Formel (II):

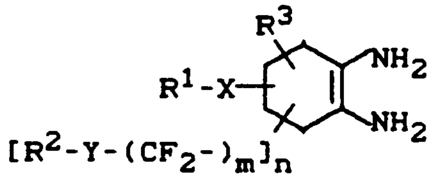

(II)

<u>Tabelle 2</u>

| Beispiel Nr. | $[R^2\text{-}Y\text{-}(CF_2\text{-})_m]_n$ | $R^1\text{-}X$ | $R^3$ | Siedepunkt (°C/mbar) | Schmelzpunkt (°C) |
|---|---|---|---|---|---|
| II-2 | 4-$OCF_3$ | 5-$OCF_3$ | H | 121/12 | - |
| II-3 | 4-$OCF_3$ | 5-$OCH_3$ | H | 130/12 | - |
| II-4 | - | 3-$OCF_3$ | H | 102/6 | 45-47 |
| II-5 | - | 5-$OCF_2CF_2H$ | 4-$CH_3$ | 120-5/0,01 | |

**Beispiel II-6:**

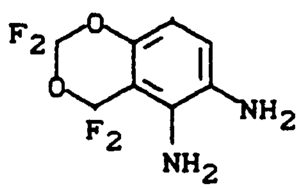

Schmelzpunkt: 50-52° C

<u>Anwendungsbeispiele</u>

In den folgenden herbiziden Tests wird die folgende Verbindung als Vergleichssubstanz eingesetzt:

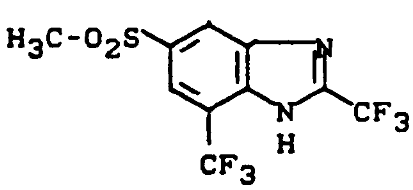

(A)

(bekannt aus DE-OS 1 642 334, Beispiel 6)

<u>Beispiel A</u>

Pre-emergence-Test

Lösungmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt z.B. die Verbindung gemäß dem Herstellungsbeispiel 7 neben einer sehr guten Nutzpflanzenverträglichkeit in Baumwolle und Weizen eine deutlich bessere herbizide Wirksamkeit gegen Unkräuter wie beispielsweise Datura, Galium, Solanum, Viola, Cynodon und Poa als die Vergleichsverbindung (A).

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebraucht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigen z.B. die Verbindungen gemäß den Herstellungsbeispielen 1 und 7 eine deutlich bessere herbizide Wirksamkeit gegen Unkräuter, wie beispielsweise Amaranthus, Datura, Ipomoea, Solanum, Panicum, und Setaria als die Vergleichssubstanz (A).

Beispiel C

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 ° C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbiespiele: 1,2 und 8.

Beispiel D

Zum Nachweis der Wirksamkeit gegen Pilze werden die minimalen Hemm-Konzentrationen (MHK) von erfindungsgemäßen Wirkstoffen bestimmt:

Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2-wöchiger Lagerung bei 28°C und 60 bis 70% rel. Luftfeuchtigkeit wird die MHK bestimmt. MHK ist die niedrigste Konzentration an Wirkstoff, bei der keinerlei Bewuchs durch die verwendete Mikrobenart erfolgt. Gute Wirkungen zeigen z.B. die Verbindungen gemäß den Herstellungsbeispielen 5 und 7 an den Testorganismen:

Alternaria tenuis
Aspergillus niger
Aureobasidium pullulans
Chaetomium globosum
Cladosporium cladosporioides
Lentinus tigrinus
Pencillium glaucum
Sclerophoma pityophila
Trichoderma viride

Beispiel E

Wirkung gegen Bakterien

Ein Agar, der als Nährmedium Bouillon enthält, wird mit erfindungsgemäßen Wirkstoffen in Konzentrationen von 1 bis 5000 ppm versetzt. Darauf infiziert man das Nährmedium jeweils mit den in Tabelle E aufgeführten Testorganismen und hält das infizierte Medium 2 Wochen bei 28°C und 60 bis 70% rel. Luftfeuchtigkeit. Die MHK ist die niedrigste Konzentration an Wirkstoff, bei der keinerlei Bewuchs durch die verwendete Mikrobenart erfolgt. Die MHK-Werte sind in Tabelle E wiedergegeben.

**Tabelle E**

**Angabe der MHK-Werte in mg/l bei der Einwirkung der unten angegebenen Wirkstoffe auf Bakterien.**

| Testorganismen | MHK in mg/l der Wirkstoffe (5) | (7) |
|---|---|---|
| Escherichia coli | 100 | <20 |
| Staphylococcus aureus | <20 | <20 |

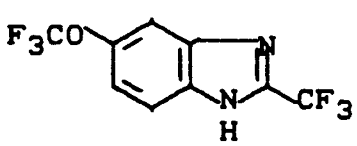
(5)

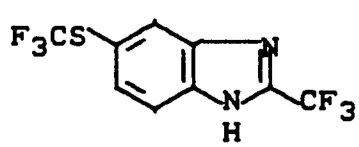
(7)

**Patentansprüche**

**1.** 2-Trifluormethyl-benzimidazole der allgemeinen Formel (I),

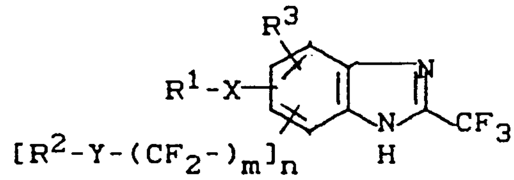

(I)

in welcher

| | |
|---|---|
| $R^1$ | für Halogenalkyl steht, |
| $R^2$ | für gegebenenfalls durch Fluor, Chlor, Brom subsituiertes Alkyl steht oder |
| $R^1$ und $R^2$ | gemeinsam für gegebenenfalls durch Fluor, Chlor, Brom substituiertes Alkylen stehen, |
| $R^3$ | für Wassestoff oder Alkyl steht, |
| X und Y | unabhängig voneinander für Sauerstoff und Schwefel stehen, |
| m | für 0 oder 1 steht und |
| n | für 0 oder 1 steht. |

**2.** 2-Trifluormethyl-benzimidazole der Formel (I) gemäß Anspruch 1, in welcher

| | |
|---|---|
| $R^1$ | für Halogenalkyl mit 1 bis Kohlenstoffatomen und 1 bis 13 gleichen oder verschiedenen Halogenatomen steht, |
| $R^2$ | für gegebenenfalls ein- oder mehrfach, gleich oder verschieden durch Fluor, Chlor, und Brom substituiertes Alkyl mit 1 bis 6 Kohlenstoffatomen steht oder |
| $R^1$ und $R^2$ | gemeinsam für gegebenenfalls ein- oder mehrfach, gleich oder verschieden durch Fluor, Chlor und Brom substituiertes Alkylen mit 1 bis 4 Kohlenstoffatomen stehen, |
| $R^3$ | für Wasserstoff oder Alkyl mit 1 bis 6 Kohlenstoffatomen steht, |
| X und Y | unabhängig voneinander für Sauerstoff und Schwefel stehen, |
| m | für 0 oder 1 steht und |
| n | für 0 oder 1 steht. |

**3.** 2-Trifluormethylbenzimidazole der Formel (I), gemäß Anspruch 1, in welcher

| | |
|---|---|
| $R^1$ | für Halogenalkyl mit 1 bis 4 Kohlenstoffatomen und 1 bis 9 gleichen oder verschiedenen Fluor-, Chlor- und Bromatomen steht, |
| $R^2$ | für gegebenenfalls ein- bis neunfach, gleich oder verschieden durch Fluor und Chlor substituiertes Alkyl mit 1 bis 4 Kohlenstoffatomen steht oder |
| $R^1$ und $R^2$ | gemeinsam für gegebenenfalls einfach bis fünffach, gleich oder verschieden durch Fluor und Chlor substituiertes Alkylen mit 1 bis 4 Kohlenstoffatomen stehen |
| $R^3$ | für Wasserstoff oder Alkyl mit 1 bis 4 Kohlenstoffatomen steht, |
| X und Y | unabhängig voneinander für Sauerstoff und Schwefel stehen, |
| m | für 0 oder 1 steht und |
| n | für 0 oder 1 steht. |

**4.** Verfahren zur Herstellung von 2-Trifluormethylbenzimidazolen der Formel (I),

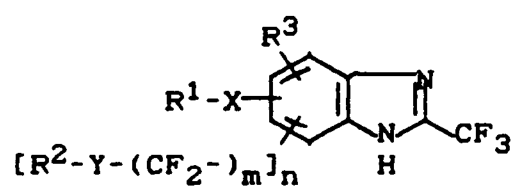

(I)

in welcher

| | |
|---|---|
| $R^1$ | für Halogenalkyl steht, |
| $R^2$ | für gegebenfalls durch Fluor-, Chlor- oder Brom substituiertes Alkyl steht oder |
| $R^1$ und $R^2$ | gemeinsam für gegebenenfalls durch Fluor-, Chlor- oder Brom substituiertes Alkylen stehen, |
| $R^3$ | für Wasserstoff oder Alkyl steht, |
| X und Y | unabhängig voneinander für Sauerstoff und Schwefel stehen, |
| m | für 0 oder 1 steht und |
| n | für 0 oder 1 steht, |

dadurch gekennzeichnet, daß man o-Phenylendiamine der Formel (II),

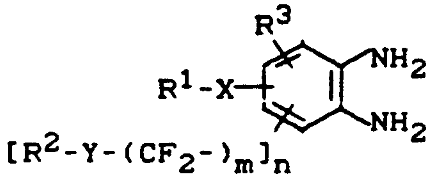

(II)

in welcher

$R^1$, $R^2$, $R^3$, X, Y, m und n die oben angegebene Bedeutung haben,

mit Trifluoressigsäure gegebenenfalls in Gegenwart von konz. Salzsäure und gegebenenfalls in Gegenwart eines Verdünnungsmittels umsetzt.

5. Herbizide und mikrobizide Mittel, gekennzeichnet durch einen Gehalt an mindestens einem 2-Trifluormethyl-benzimidazol der Formel (I) gemäß den Ansprüchen 1 bis 4.

6. Verfahren zur Bekämpfung von Umkräutern, dadurch gekennzeichnet, daß man 2-Trifluormethyl-benzimidazole der Formel (I) gemäß den Ansprüchen 1 bis 4 auf die Unkräuter und/oder ihren Lebensraum einwirken läßt.

7. Verfahren zur Bekämpfung von Mikroben, dadurch gekennzeichnet, daß man 2-Trifluormethyl-benzimidazole der Formel (I) gemäß den Ansprüchen 1 bis 4 auf Mikroben und/oder ihren Lebensraum einwirken läßt.

8. Verwendung von 2-Trifluormethyl-benzimidazolen der Formel (I) gemäß den Ansprüchen 1 bis 4 zur Bekämpfung von Unkräuten und Mikroben.

9. Verfahren zur Herstellung von herbiziden und mikrobiziden Mitteln, dadurch gekennzeichnet, daß man 2-Trifluormethyl-benzimidazole der Formel (I) gemäß den Ansprüchen 1 bis 4 mit Streckmitteln und/oder oberflächenaktiven Substanzen vermischt.

## Claims

1. 2-Trifluoromethyl-benzimidazoles of the general formula (I)

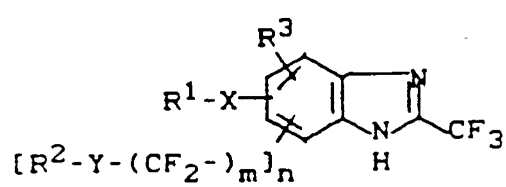

(I)

in which

| | |
|---|---|
| $R^1$ | represents halogenoalkyl, |
| $R^2$ | represents alkyl which is optionally substituted by fluorine, chlorine or bromine, or |
| $R^1$ and $R^2$ | together represent alkylene, which is optionally substituted by fluorine, chlorine or bromine |
| $R^3$ | represents hydrogen or alkyl, |
| X and Y | independently of one another represent oxygen and sulphur, |
| m | represents 0 or 1 and |
| n | represents 0 or 1. |

2. 2-Trifluoromethyl-benzimidazoles of the formula (I) according to Claim 1, in which

| | |
|---|---|
| $R^1$ | represents halogenoalkyl having 1 to 6 carbon atoms and 1 to 13 identical or different halogen atoms, |
| $R^2$ | represents alkyl having 1 to 6 carbon atoms which is optionally monosubstituted or polysubstituted by identical or different substituents from the series consisting of |

| | |
|---|---|
| | fluorine, chlorine and bromine, or |
| $R^1$ and $R^2$ | together represent alkylene having 1 to 4 carbon atoms which is optionally monosubstituted or polysubstituted by identical or different substituents from the series consisting of fluorine, chlorine and bromine. |
| $R^3$ | represents hydrogen or alkyl having 1 to 6 carbon atoms, |
| X and Y | independently of one another represent oxygen and sulphur, |
| m | represents 0 or 1 and |
| n | represents 0 or 1. |

**3.** 2-Trifluoromethyl-benzimidazoles of the formula (I) according to Claim 1, in which

| | |
|---|---|
| $R^1$ | represents halogenoalkyl having 1 to 4 carbon atoms and 1 to 9 identical or different fluorine, chlorine or bromine atoms, |
| $R^2$ | represents alkyl having 1 to 4 carbon atoms which is optionally monosubstituted to nonasubstituted by identical or different substituents from the series consisting of fluorine and chlorine, or |
| $R^1$ and $R^2$ | together represent alkylene having 1 to 4 carbon atoms which is optionally monosubstituted to pentasubstituted by identical or different substituents from the series consisting of fluorine and chlorine, |
| $R^3$ | represents hydrogen or alkyl having 1 to 4 carbon atoms, |
| X and Y | independently of one another represent oxygen and sulphur, |
| m | represents 0 or 1 and |
| n | represents 0 or 1. |

**4.** Process for the preparation of 2-trifluoromethyl-benzimidazoles of the formula (I)

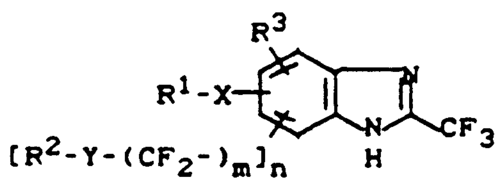

(I)

in which

| | |
|---|---|
| $R^1$ | represents halogenoalkyl, |
| $R^2$ | represents alkyl which is optionally substituted by fluorine, chlorine or bromine, or |
| $R^1$ and $R^2$ | together represent alkylene, which is optionally substituted by fluorine, chlorine or bromine |
| $R^3$ | represents hydrogen or alkyl, |
| X and Y | independently of one another represent oxygen and sulphur, |
| m | represents 0 or 1 and |
| n | represents 0 or 1, |

characterised in that o-phenylenediamines of the formula (II)

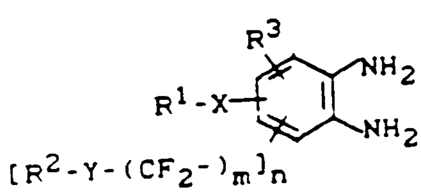

(II)

in which

$R^1$, $R^2$, $R^3$, X, Y, m and n have the meaning given above,

are reacted with trifluoroacetic acid, if appropriate in the presence of concentrated hydrochloric acid and, if appropriate, in the presence of a diluent.

**5.** Herbicidal and microbicidal agents, characterised in that they contain at least one 2-trifluoromethyl-benzimidazole of the formula (I) according to Claims 1 to 4.

6. Method of combating weeds, characterised in that 2-trifluoromethyl-benzimidazoles of the formula (I) according to Claims 1 to 4 are allowed to act on the weeds and/or their habitat.

7. Method of combating microbes, characterised in that 2-trifluoromethyl-benzimidazoles of the formula (I) according to Claims 1 to 4 are allowed to act on microbes and/or their habitat.

8. Use of 2-trifluoromethyl-benzimidazoles of the formula (I) according to Claims 1 to 4 for combating weeds and microbes.

9. Process for the preparation of herbicidal and microbicidal agents, characterised in that 2-trifluoromethyl-benzimidazoles of the formula (I) according to Claims 1 to 4 are mixed with extenders and/or surface-active substances.

**Revendications**

1. 2-trifluorométhylbenzimidazoles de formule générale (I)

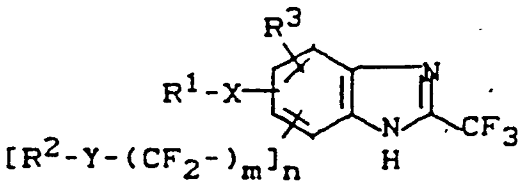

(I)

dans laquelle

$R^1$ représente un halogénoalkyle,
$R^2$ représente un groupe alkyle éventuellement substitué par le fluor, le chlore ou le brome, ou
$R^1$ et $R^2$ représentent ensemble un groupe alkylène éventuellement substitué par le fluor, le chlore ou le brome,
$R^3$ représente l'hydrogène ou un groupe alkyle,
X et Y représentent indépendamment d'un de l'autre l'oxygène et le soufre,
m signifie 0 ou 1, et
n signifie 0 ou 1.

2. 2-trifluorométhylbenzimidazoles de formule générale (I) selon la revendication 1, dans laquelle
$R^1$ représente un halogénoalkyle avec 1 à 6 atomes de carbone et 1 à 13 atomes d'halogène identiques ou différents,
$R^2$ représente un groupe alkyle avec 1 à 6 atomes de carbone, éventuellement substitué une ou plusieurs fois, de manière identique ou différente, par le fluor le chlore et le brome, ou
$R^1$ et $R^2$ représentent ensemble un groupe alkylène avec 1 à 4 atomes de carbone, éventuellement substitués une ou plusieurs fois de manière identique ou différente par le fluor, le chlore et le brome,
$R^3$ représente l'hydrogène ou un groupe alkyle avec 1 à 6 atomes de carbone,
X et Y représentent indépendamment l'un de l'autre l'oxygène et le soufre,
m signifie 0 ou 1, et
n signifie 0 ou 1.

3. 2-trifluorométhylbenzimidazoles de formule générale (I) selon la revendication 1, dans laquelle
$R^1$ représente un halogénoalkyle avec 1 à 4 atomes de carbone et 1 à 9 atomes de fluor, de chlore et de brome identiques ou différents,
$R^2$ représente un groupe alkyle avec 1 à 4 atomes de carbone, éventuellement substitué 1 à 9 fois, de manière identique ou différente, par le fluor et le chlore, ou
$R^1$ et $R^2$ représentent ensemble un groupe alkylène avec 1 à 4 atomes de carbone, éventuellement substitué 1 à fois de manière identique ou différente par le fluor et le chlore,
$R^3$ représente l'hydrogène ou un groupe alkyle avec 1 à 4 atomes de carbone,
X et Y représentent indépendamment l'un de l'autre l'oxygène et le soufre,
m signifie 0 ou 1, et
n signifie 0 ou 1.

4. Procédé pour la fabrication des 2-trifluorométhylbenzimidazoles de formule (I),

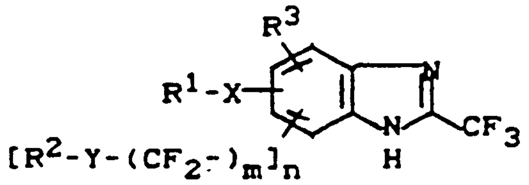

(I)

dans laquelle
$R^1$ représente un halogénoalkyle,
$R^2$ représente un groupe alkyle éventuellement substitué par le fluor, le chlore ou le brome,
$R^1$ et $R^2$ représentent ensemble un groupe alkylène éventuellement substitué par le fluor, le chlore ou le brome,
$R^3$ représente l'hydrogène ou un groupe alkyle,
X et Y représentent indépendamment d'un de l'autre l'oxygène et le soufre,
m signifie 0 ou 1, et
n signifie 0 ou 1,
caractérisé en ce que l'on fait réagir des o-phénylènediamines de formule (II)

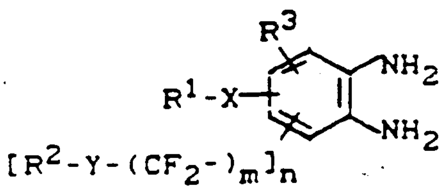

(II)

dans laquelle $R^1$, $R^2$, $R^3$, X, Y, m et n possèdent les significations décrites ci-dessus,
avec de l'acide trifluoracétique, éventuellement en présence d'acide chlorhydrique concentré et éventuellement en présence d'un diluant.

5. Agents herbicides et microbicides caractérisés en ce qu'ils contiennent au moins un 2-trifluorométhylbenzimidazole de formule (I) selon les revendications 1 à 4.

6. Procédé pour la lutte contre les mauvaises herbes, caractérisé en ce qu'on fait agir des 2-trifluorométhylbenzimidazoles de formule (I) selon les revendications 1 à 4 sur les mauvaises herbes et/ou sur leur habitat.

7. Procédé pour la lutte contre les microbes, caractérisé en ce que l'on fait agir des 2-trifluorométhylbenzimidazoles de formule (I) selon les revendications 1 à 4 sur les microbes et/ou sur leur habitat.

8. Utilisation des 2-trifluorométhylbenzimidazoles de formule (I) selon les revendications 1 à 4 pour lutter contre les mauvaises herbes et les microbes.

9. Procédé pour la fabrication d'agents herbicides et microbicides, caractérisé en ce que l'on mélange des 2-trifluorométhylbenzimidazoles de formule (I) selon les revendications 1 à 4 avec des agents d'allongement et/ou des substances tensio-actives.